# EUROPÄISCHE PATENTSCHRIFT

(11) **EP 3 638 144 B1**
(45) Veröffentlichungstag und Bekanntmachung des Hinweises auf die Patenterteilung: **01.01.2025**
(21) Anmeldenummer: 18728357.7
(22) Anmeldetag: 30.05.2018
(51) Int. Cl.: A61L 2/26, A61B 50/20, A61B 50/22, A61B 50/30, A61B 50/33, A61B 50/34, A61B 90/90, A61L 2/24, G16H 30/40, G16H 40/40, G16H 40/60, A61L 2/07

(54) **INSTRUMENTENSIEB FÜR CHIRURGISCHE INSTRUMENTE**
INSTRUMENT TRAY FOR SURGICAL INSTRUMENTS
BOÎTE À INSTRUMENTS POUR DES INSTRUMENTS CHIRURGICAUX

(30) Priorität: 13.06.2017 DE 102017209966
(43) Veröffentlichungstag der Anmeldung: 22.04.2020
(73) Patentinhaber: KUKA Deutschland GmbH, 86165 Augsburg (DE)
(72) Erfinder: KEIBEL, Andreas, 86161 Augsburg (DE)
(74) Vertreter: Oelke, Jochen
(86) Internationale Anmeldenummer: PCT/EP2018/064128
(87) Internationale Veröffentlichungsnummer: WO 2018/228818

(56) Entgegenhaltungen:
- DE-A1- 102004 053 355
- US-A1- 2011 005 342
- US-A1- 2014 069 841
- US-A1- 2014 083 886

## Beschreibung

### 1. Technisches Gebiet

Die vorliegende Erfindung betrifft ein System und Verfahren zur zumindest teilweise automatischen Handhabung eines Instrumentensiebs zur Aufbewahrung und Bereitstellung von chirurgischen Instrumenten.

### 2. Technischer Hintergrund

Chirurgische Instrumente (auch als Operationsbesteck bzw. Operationsinstrumente bezeichnet), wie sie typischerweise in Operationssälen in Kliniken verwendet werden, werden vornehmlich wiederverwendet und müssen daher in einem Kreislaufprozess zwischen Operationssaal und Sterilisationsabteilung pendeln. In diesem Kreislauf werden die Instrumente vielfach manuell angefasst und mehrfach in verschiedene Behältnisse umgelagert. Diese Prozesse sind bisher kaum automatisierbar, da die Instrumente typischerweise ungeordnet in Instrumentensieben bzw. -körben nach der Benutzung im Operationssaal abgelegt werden. Die Handhabung dieser Instrumentensiebe und der darin enthaltenen Instrumente ist daher sehr arbeitsintensiv und dementsprechend teuer.

Der Ablauf eines typischen Instrumentenkreislaufs in einer Klinik ist beispielhaft wie folgt. Im Operationssaal werden die benutzten chirurgischen Instrumente unsystematisch in so genannten Instrumentensieben (wie etwa Drahtkörbe oder andere Behältnisse, die Öffnungen aufweisen, um ein Spülen der darin enthaltenen Instrumente zu ermöglichen) gesammelt. Verbrauchs- und Einwegmaterialien werden weggeworfen. Anschließend werden die benutzten Instrumente zur Sterilisationsabteilung transportiert. In der Sterilisationsabteilung werden die Instrumentensiebe ausgepackt und eine manuelle Vorinspektion durchgeführt. Dabei werden einzelne Instrumente bereits vorgereinigt, beispielsweise mittels Ultraschall. Anschließend werden die Siebe manuell für die Reinigungs- und Desinfektionsgeräte zusammengestellt und die Instrumente anschließend gespült. Die gespülten und so gereinigten Instrumente kommen in ein Lager, wo sie bis kurz vor der nächsten Verwendung aufbewahrt werden. Für eine Operation werden die benötigten Instrumente individuell angefragt. Die angefragten Instrumente werden aus dem Lager abgeholt und zu Packtischen transportiert, wo die Instrumentensiebe manuell für die jeweilige Operation gepackt bzw. bestückt werden. Dieser Arbeitsschritt ist sehr aufwendig und personalintensiv. Nach einer visuellen Nachinspektion werden die so gepackten Siebe in Steriltuch eingepackt und beispielsweise zu einem Autoklaven transportiert. Dort werden sie sterilisiert und nach dem Sterilisationsvorgang entladen und zum OP-Bereich transportiert. Gegebenenfalls werden sogenannte chirurgische Case-Carts mit den vorbereiteten und sterilisierten Instrumentensieben vorbereitet und in den Operationssaal verbracht. Dort werden die Case-Carts ausgepackt und die sterilisierten Instrumente auf den Instrumententischen neben dem Operationstisch ausgebreitet, sodass die Instrumente griffbereit sind.

Die US2014/0083886 A1 offenbart ein modulares Instrumentensieb, bei dem Instrumentenhalterungen mittels separaten Federstiften in vorgegebene Lochpositionen des Instrumentensiebs und den Instrumentenhalterungen eingebracht und befestigt werden können.

Die US 2014/0069841 A1 offenbart ebenfalls ein modulares Instrumentensieb mit universellen Halterungen zur Befestigung von Instrumenten in dem Instrumentensieb.

Die US 2011/0005342 A1 offenbart ein robotisches System zur automatisierten Ent- und Beladung eines Instrumentenhalters mit sterilisierbaren Instrumenten, wobei die Instrumente durch den Roboter gegriffen und mittels einer Kamera erfasst werden.

Ziel der Erfindung ist es, den Aufwand des Kreislaufs des Operationsbestecks bzw. der chirurgischen Instrumente zu reduzieren und damit die Kosten und Zeitdauer eines Instrumentenzyklus zu reduzieren. Insbesondere soll die Handhabung der Instrumentensiebe vereinfacht und vorzugsweise zumindest teilweise automatisiert werden. Diese und weitere Aufgaben werden durch ein System gemäß Anspruch 1, sowie ein Verfahren nach Anspruch 9 zumindest teilweise gelöst.

### 3. Inhalt der Erfindung

Die vorliegende Erfindung betrifft ein System umfassend ein Instrumentensieb, sowie einen Manipulator, der eingerichtet ist zur Bestückung dieses Instrumentensiebs mit chirurgischen Instrumenten und/oder zur Entnahme von Instrumenten aus dem Instrumentensieb.

Das Instrumentensieb zur Aufbewahrung und Bereitstellung von chirurgischen Instrumenten, umfassend einen Behälter zur Aufnahme von chirurgischen Instrumenten, wobei der Behälter mit eindeutigen visuellen Markern für individuelle Instrumente versehen ist. Damit können Instrumenten eindeutige Instrumenten-Positionen im Behälter zugeordnet werden. Unter einem Instrumentensieb wird hierin jede Art von Vorrichtung verstanden, die zur Aufnahme von chirurgischen Instrumenten geeignet ist und über entsprechende Ablauföffnungen verfügt, die ein Spülen des Instrumentensiebs mit darin enthaltenen Instrumenten erlauben. Beispielhaft kann das Instrumentensieb einen Drahtkorb aus Metall umfassen, in den die Instrumente eingelegt werden und der zusammen mit den Instrumenten in eine entsprechend geeignete Spülvorrichtung einführbar ist. Die Reinigungs- und Desinfektionsmittel können damit einfach ablaufen, sodass das Instrumentensieb zusammen mit den gereinigten Instrumenten in einem Stück aus der Spülvorrichtung entnommen werden kann. Der Behälter zur Aufnahme der Instrumente weist für jedes individuelle Instrument eine eindeutige Position auf, die durch den Marker definiert wird. Die Instrumente werden also nicht lose und ungeordnet im Behälter aufgenommen, sondern jedes Instrument hat seinen eigenen, und eindeutig zugeordneten Platz im Behälter. Dies bietet unter anderem den Vorteil, dass die Instrumente beispielsweise komplett in dieser Form im Behälter im Operationssaal bereitgestellt werden können und nicht länger vor der Operation manuell aus dem Behälter entnommen und auf dem Operationstisch bereitgestellt werden. Alle Instrumente liegen an fest definierten und markierten Stellen, sodass eine manuelle Anordnung der Instrumente auf den Instrumententischen neben dem Operationstisch nicht länger notwendig ist. Besonders vorteilhaft ist es, wenn diese Instrumentensiebe im Ganzen, also mit den vollständig bestückten Instrumenten, sterilisierbar sind. Die bestückten und sterilisierten Instrumentensiebe verbleiben dann in diesem Zustand und müssen nicht wieder umgepackt werden. Weiter ist es grundsätzlich vorteilhaft, wenn die Instrumentensiebe in etwa die Größe der Instrumententische haben. Damit können die Instrumente im Operationssaal auf den Instrumententischen bereitgestellt werden, jedoch ohne das es notwendig wäre, die Instrumente manuell aus dem Sieb zu entnehmen. Stattdessen kann der operierende Arzt oder ein Assistent die Instrumente wie benötigt während der Operation direkt aus dem Sieb entnehmen. Weiter sind die Marker vorteilhaft lösbar befestigt, so dass die Instrumenten-Positionen, sowie ggf. die Art der Instrumente, frei und individuell angepasst werden können.

Vorzugsweise sind die visuellen Marker Silhouetten der individuellen Instrumente. Dabei weisen diese besonders bevorzugt die gleiche Größe auf wie die Instrumente, die sie repräsentieren bzw. darstellen. Jede Silhouette befindet sich genau an der Stelle, an der das jeweilige Instrument positioniert sein soll. Auf diese Weise sind Leerstellen sofort sichtbar und Fehlbestückungen sofort und ohne weiteres erkennbar. Die Verwendung von eindeutigen visuellen Markern, wie etwa die genannten Silhouetten, ermöglicht insbesondere, dass eine automatisierte Handhabung der erfindungsgemäßen Instrumentensiebe möglich ist. Aufgrund der Marker ist es nämlich möglich, bspw. anhand von computergestützter Bildverarbeitung, den Bestückungszustand eines Instrumentensiebs einfach und sicher zu bestimmen. Mit Instrumentensieben des Standes der Technik ist dies nicht möglich, da dort eine Vielzahl von Instrumenten wahllos und übereinander angeordnet werden. Die Verwendung von Silhouetten der individuellen Instrumente als Marker vereinfacht eine sichere automatisierte Bilderfassung und Verarbeitung der Instrumentensiebe. Besonders auch für die manuelle Sichtkontrolle oder ggf. manuelle Nachbestückung sind die Silhouetten ein wertvolles Hilfsmittel, mit dem die Qualität und Effizienz im Instrumentenkreislauf gesteigert werden kann.

Vorzugsweise weist der Behälter Halteklemmen auf, die eingerichtet sind, um die Instrumente lösbar und mit Spiel zu halten. Die Instrumente sollten insbesondere nicht zu fest gehalten werden, da sie gegebenenfalls während einer Operation einfach und schnell entnommen werden müssen. Die Halterung mit Spiel ist zudem vorteilhaft, damit die Instrumente bei einem Reinigungsvorgang vollständig von den Reinigungsflüssigkeiten benetzt werden können.

Besonders bevorzugt sind die Halteklemmen mit Befestigungsstiften versehen, und der Behälter weist Öffnungen auf, durch die die Stifte geführt werden können. Die Stifte sind dabei zur Fixierung der Halteklemmen am Behälter verformbar eingerichtet. Derartige Halteklemmen erlauben eine individuelle Bestückung der Instrumentensiebe, indem sie, zusammen mit den visuellen Markern, frei an dem Behälter positioniert und fixiert werden können. Hierzu werden die Stifte durch entsprechende Öffnungen im Behälter geführt, wie etwa Öffnungen in einem Gitterkorb, wenn der Behälter als Gitterkorb ausgebildet ist, und anschließend werden die Stifte an der Unterseite des Behälters umgebogen, wodurch die Halteklemmen am Behälter fixiert werden. Dies kann gegebenenfalls in der Klinik selbst vorgenommen werden, da die Stifte gegebenenfalls mit einer einfachen Zange umgebogen werden können.

Vorzugsweise weisen die Halteklemmen jeweils zwei Federarme auf, die eingerichtet sind, um zwischen sich einen Teil eines Instruments lösbar und mit Spiel haltend aufzunehmen. Die Federn sind dabei derart zueinander angeordnet, dass sie eine Schnappverbindung mit den zu haltenden Instrumenten eingehen. Hierdurch ist ein einfaches Bestücken des Behälters mit Instrumenten möglich und dennoch eine einfache und komfortable Entnahme der Instrumente aus dem Behälter, beispielsweise während einer Operation, gewährleistet.

Generell bevorzugt ist das Instrumentensieb autoklavierbar. Alle Materialien des Instrumentensiebs sind also vorzugsweise derart gewählt, dass das Instrumentensieb ohne weitere Umbauten direkt in einen Autoklaven eingesetzt und dort sterilisiert werden kann. Vorzugsweise besteht das Instrumentensieb aus Metall.

Das Instrumentensieb weist Kopplungsvorrichtungen auf, die eingerichtet sind, um das Instrumentensieb in einem chirurgischen Case-Cart anzuordnen. Auf diese Weise ist ein sicherer Transport der Instrumentensiebe gewährleistet.

Generell bevorzugt sind die Instrumenten-Positionen im Behälter in einer Ebene angeordnet, sodass sich die Instrumente im Behälter nicht bzw. nicht wesentlich überlappen. Dies ist im Unterschied zum Stand der Technik, wo die Instrumente vor einer OP dicht gepackt bzw. gebündelt oder gestapelt in kleinen Körben bereitgestellt werden, oder (z. B. nach der OP) in den Körben wahllos angeordnet sind respektive wahllos in die Körbe hineingeworfen werden. Durch die Anordnung in einer Ebene, also nebeneinander, ist es nunmehr möglich, die Instrumente automatisiert beispielsweise mittels eines geeigneten Manipulators zu handhaben. Zudem erlaubt die Anordnung in einer Ebene die direkte Verwendung des bestückten Instrumentensiebs im Operationssaal. Es ist nicht länger notwendig die Instrumente aus dem Sieb zu entnehmen und bereitzustellen, da das Instrumentensieb selbst bereits eine geeignete Bereitstellung bietet. Dadurch verkürzt sich die OP-Vorbereitungszeit erheblich.

Wie oben dargelegt erlaubt das erfindungsgemäße Instrumentensieb die Automatisierung des Bestückungs- bzw. Handhabungsvorgangs des Siebs. Da jedes Instrument eine eindeutige Instrumenten-Position im Sieb hat, ist es mit den erfindungsgemäßen Sieben möglich, die darin enthaltenen Instrumente beispielsweise teil- oder vollautomatisiert mittels eines Manipulators zu handhaben.

Manipulatoren, und insbesondere Roboter, sind programmierbare Maschinen, die insbesondere zur automatischen Handhabung oder Bearbeitung von Gegenständen bzw. Werkstücken ausgebildet sind. Ein typischer Vertreter solcher Manipulatoren sind sogenannte Gelenkarmroboter, die über eine Vielzahl von Gliedern verfügen, die wiederum über entsprechende Gelenke miteinander verbunden sind. An einem freien Ende ist typischerweise eine Aufnahme für ein Werkzeug vorgesehen.

Das System umfasst weiter eine Bilderkennungseinheit mit einer Kamera zur Bilderfassung und eine Einrichtung zur Bildverarbeitung, wobei die Bilderkennungseinheit der Steuereinrichtung des Manipulators Informationen über den Bestückungszustand des Instrumentensiebs übermittelt. Die Steuereinrichtung ist eingerichtet, um den Manipulator unter Verwendung der Informationen zur Bestückung und/oder Entnahme von Instrumenten aus dem Instrumentensieb anzusteuern. Das System erlaubt es somit eine automatisierte Erfassung des Bestückungszustands und eine automatisierte Ansteuerung des Manipulators, um beispielsweise falsch positionierte Instrumente zu erkennen und automatisiert richtig anzuordnen, um fehlende Instrumente zu ersetzen oder aber um gezielt bestimmte Instrumente aus dem Instrumentensieb zu entnehmen.

Das System umfasst weiter ein chirurgisches Case-Cart, welches zur Aufnahme von Instrumentensieben geeignet ist, sowie ein fahrerloses Transportfahrzeug (auch automatisches Flurförderfahrzug genannt - englisch: automated guided vehicle, AGV) zum Bewegen des Case-Carts. Fahrerlose Transportfahrzeuge sind flurgebundene Fördermittel mit eigenem Fahrantrieb, die automatisch gesteuert und berührungslos geführt werden. Die Verwendung derartiger Fördermittel kann die Automatisierung der Handhabung der Instrumentensiebe weiter vereinfachen.

Weiter umfasst das System eine Sterilisationsvorrichtung, wobei die Steuereinrichtung des Manipulators eingerichtet ist, um bei Erkennung eines vollen oder im Wesentlichen vollen Bestückungszustands das Instrumentensieb zu greifen und in die Sterilisationsvorrichtung zu bewegen und nach Abschluss des Sterilisationsvorgangs wieder zu entnehmen. Die Erkennung des Bestückungszustands wird durch die besonderen Merkmale des erfindungsgemäßen Instrumentensiebs vereinfacht, sodass ein sicheres Erkennen und Handhaben des Siebs durch einen Manipulator möglich ist.

Generell ist es vorteilhaft, wenn das Instrumentensieb mit eindeutigen, maschinenlesbaren Codes versehen ist. Auf diese Weise ist es möglich verschiedene Siebe eindeutig zu identifizieren, was eine automatisierte Handhabung vereinfacht.

Die Erfindung betrifft auch ein Verfahren zum zumindest teilweise automatisierten Handhaben eines Instrumentensiebs des Systems wie hierin beschrieben, aufweisend folgende Schritte: Bereitstellen eines erfindungsgemäßen Instrumentensiebs; Bereitstellen eines Manipulators mit einer zugeordneten Bilderkennungseinheit, umfassend eine Kamera zur Bilderfassung und eine Einrichtung zur Bildverarbeitung; Erfassen eines Bildes des Instrumentensiebs und Erkennen des Bestückungszustands mittels der Bilderkennungseinheit; Ansteuern des Manipulators unter Berücksichtigung des Bestückungszustands zur Bestückung und/oder Entnahme von Instrumenten des Instrumentensiebs.

Aufgrund der vorgegebenen Instrumenten-Positionen in dem erfindungsgemäßen Instrumentensieb ist eine Automatisierung mit Manipulatoren und der Hilfe von Bildverarbeitung möglich. Beispielsweise können Instrumentensiebe, die Fehlteile aufweisen, nun automatisch durch die Bildverarbeitung erkannt und gegebenenfalls vervollständigt bzw. richtiggestellt werden. Eine robotische Bestückung der Instrumentensiebe ist durch die genau vorgegebenen Instrumentenpositionen vorteilhaft möglich. Dabei unterstützt die Integration von Robotern die Sicherheit der Sterilisation, da Roboter keimfrei betrieben werden können. Die Einrichtung zur Bildverarbeitung kann bspw. ein hierfür speziell eingerichteter Computer sein, oder sie kann in der Manipulatorsteuerung integriert sein, oder sich Hardware-Komponenten mit dieser teilen.

Vorzugsweise erfolgt die Ansteuerung des Manipulators im vorliegenden Verfahren daher derart, dass der Manipulator leere Instrumentenpositionen bestückt. Dabei müssen nicht zwingend alle leeren Position bestückt werden, sondern es ist ebenfalls denkbar, dass der Manipulator nur bestimmte, vorgegebene Positionen bestückt.

Weiter bevorzugt erfolgt die Ansteuerung des Manipulators im Verfahren derart, dass der Manipulator auf Anweisung eines Anwenders ausgewählte Instrumente entnimmt. Dies kann beispielsweise während einer Operation geschehen, wenn ein Roboter die Instrumente dem Chirurgen anreicht.

Bei Erkennen eines vollen oder im Wesentlichen vollen Bestückungszustands werden folgende Schritte ausgeführt: Ergreifen des Instrumentensiebs durch den Manipulator; Bewegen des Instrumentensiebs mittels des Manipulators in eine Sterilisationsvorrichtung; Durchführen eines Sterilisationsvorgangs in der Sterilisationsvorrichtung; und danach Entnahme des sterilisierten Instrumentensiebs mittels des Manipulators aus der Sterilisationsvorrichtung. Die Bildverarbeitung ermöglicht es, dass das System im vorliegenden Verfahren automatisch erkennt, ob beispielsweise ein Instrumentensieb vollständig bestückt ist bzw. eine Bestückung gemäß spezifischer Vorgaben aufweist und der Manipulator kann in diesem Fall das Sieb automatisiert einer Sterilisationsvorrichtung zuführen. Da das sterilisierte Sieb nicht manuell durch einen Menschen gehandhabt werden muss, sondern durch den Manipulator entnommen werden kann, bleibt das Sieb steril.

Nach der Entnahme des sterilisierten Instrumentensiebs mittels des Manipulators werden weiter folgende Schritte durchgeführt: Anordnen des Instrumentensiebs mittels des Manipulators in einem chirurgischen Case-Cart; und Bewegen des Case-Carts mittels eines fahrerlosen Transportfahrzeugs zu einem Operationssaal. Das Instrumentensieb ist derart eingerichtet, dass es in einem chirurgischen Case-Cart angeordnet werden kann. Zur automatisierten Handhabung ist zudem vorzugsweise ein maschinenlesbarer Code (wie z.B. ein Strichcode, QR-Code, Spaltgitter, etc.) an jedem Instrumentensieb an geeigneten Positionen vorgesehen. Der Code erlaubt zudem vorteilhaft eine eindeutige Identifizierung des Siebs, wozu beispielsweise entsprechende Informationen in einer Datenbank hinterlegt werden können. Das Case-Cart verfügt vorteilhaft über eine entsprechende Deichsel zur Anbindung an das Transportfahrzeug, damit das fahrerlose Transportfahrzeug an das Case-Cart ankoppeln und dieses bewegen kann. Hierzu kann das Case-Cart beispielsweise über maschinenlesbare Markierungen verfügen, sodass ein mit einer Bilderkennung ausgestattetes Transportfahrzeug automatisch erkennen kann, welches Case-Cart bewegt werden soll.

### 4. Beschreibung bevorzugter Ausführungsbeispiele

Im Folgenden werden bevorzugte Ausführungsformen der Erfindung unter Bezugnahme auf die Zeichnungen im Detail erläutert. Hierbei zeigt:
- Figur 1: eine schematische Draufsicht eines Instrumentensiebs;
- Figur 2a: eine schematische, geschnittene Seitenansicht des Instrumentensiebs von Figur 1;
- Figur 2b: eine schematische Detaildarstellung einer Halteklemme; und
- Figur 3: eine schematische Darstellung eines Systems zur automatisierten Handhabung von Instrumentensieben.

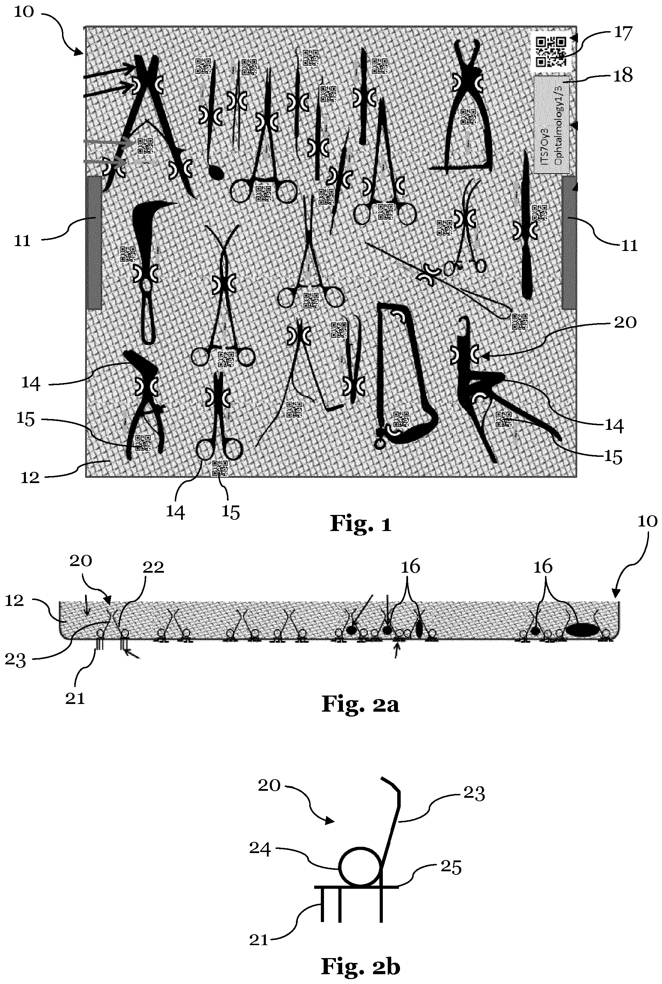

Figur 1 zeigt ein Instrumentensieb 10 zur Aufbewahrung und Bereitstellung von chirurgischen Instrumenten. Das System umfasst einen Behälter 12 zur Aufnahme der Instrumente, der in Form eines Drahtkorbs vorliegt. Der Behälter 12 muss nicht zwingend ein Korb oder ein Geflecht aufweisen, wesentlich ist vielmehr, dass der Boden des Behälters perforiert ist, sodass gegebenenfalls Reinigungsflüssigkeit oder Sterilisationsflüssigkeit abfließen kann. Der Boden des Behälters 12 ist mit einer Reihe von eindeutigen visuellen Markern 14 versehen. Die Marker 14 sind im gezeigten Ausführungsbeispiel Silhouetten der individuellen Instrumente, die an der angegebenen Position befestigt werden sollen. Die Marker bzw. Silhouetten 14 weisen die gleiche Form und Größe auf wie die Instrumente, die sie repräsentieren. Die in Figur 1 schematisch dargestellten chirurgischen Instrumente sollen also nicht die Instrumente selbst darstellen, sondern nur Silhouetten der Instrumente. Jeder Silhouette bzw. jedem Marker 14 ist zusätzlich ein individueller maschinenlesbarer Code 15 zugeordnet, mit dem das zugeordnete Instrument ebenfalls eindeutig identifiziert werden kann. Auch das Instrumentensieb 10 weist einen maschinenlesbaren Code 17 auf, mit dem das Sieb eindeutig identifiziert werden kann. Neben dem Code 17 ist eine alphanumerische Bezeichnung 18 für das Sieb angeordnet. Die Bezugszeichen 11 bezeichnen Kopplungsvorrichtungen, mit denen das Sieb beispielsweise in einem chirurgischen Case-Cart angeordnet werden kann. Die Kopplungsvorrichtungen 11 können auch als Griffe für einen Manipulator oder einen menschlichen Anwender dienen. Vorteilhaft sind alle Instrumenten-Positionen im Behälter 12 in einer Ebene angeordnet, sodass sich die Instrumente im Behälter nicht überlappen. Jeder Instrumenten-Position, die durch einen Marker bzw. eine Silhouette 14 festgelegt wird, sind ein oder mehrere Halteklemmen 20 zugeordnet, mit denen die jeweiligen Instrumente lösbar und mit Spiel gehalten werden können.

In Figur 2a ist das Sieb aus Figur 1 in einer schematischen, geschnittenen Seitenansicht gezeigt. Die Bezugszeichen 16 bezeichnen verschiedene chirurgische Instrumente (von denen in der Figur nur schematische Querschnitte zu sehen sind), die von den Halteklemmen 20 gehalten sind. In dem gezeigten Ausführungsbeispiel (siehe auch Figur 2b) umfasst jede Halteklemme zwei Federarme 22, 23, welche mittels Federn 24 elastisch federnd an einer Basis 25 vorgesehen sind. Die Basis 25 ist mit ein oder mehreren Befestigungsstiften 21 versehen, die durch Öffnungen im Boden des Behälters 12 eingesteckt werden können, sodass sich die Befestigungsstifte 21 aus der Unterseite des Behälters 12 erstrecken. Dies ist in Fig. 2b bei der linken Befestigungsklemme gezeigt. Die Befestigungsstifte 21 sind verformbar und können umgebogen werden, um die Halteklemmen am Boden des Behälters 12 zu fixieren. Dies ist in Fig. 2b bei den übrigen Befestigungsklemmen gezeigt. Auf diese Weise können die Halteklemmen individuell im Behälter 12, wie benötigt, eingerichtet und fixiert werden.

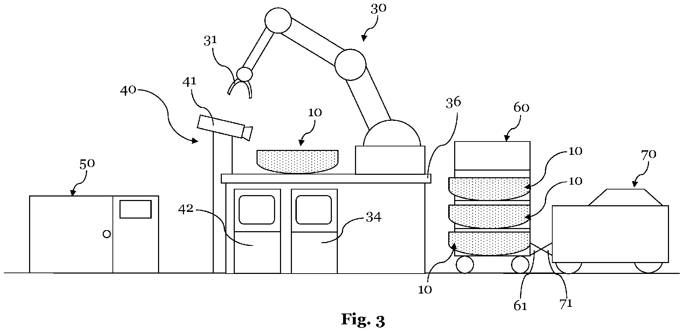

Figur 3 ist eine schematische Ansicht eines Systems zur teil- bzw. vollautomatischen Handhabung von Instrumentensieben 10. Das System umfasst einen Manipulator 30, der in Form eines Gelenkarmroboters vorgesehen ist. Der Manipulator 30 trägt einen Greifer 31, mit dem chirurgische Instrumente gegriffen werden können. Auf einem Tisch 36 ist ein Instrumentensieb 10 bereitgestellt. Eine Bilderkennungseinheit 40 mit einer Kamera 41 zur Bilderfassung und einer Einrichtung zur Bildverarbeitung 42 ist so angeordnet, dass sie Bilder vom Inneren des Instrumentensiebs 10 erfassen kann. Die Bilderkennungseinheit 40 ist dabei eingerichtet, um einer Steuereinrichtung 34 des Manipulators 30 Informationen über beispielsweise den Bestückungszustand des Instrumentensiebs 10 zu übermitteln. Die Steuereinrichtung 34 des Manipulators 30 kann dann wiederum den Manipulator 30 entsprechend ansteuern, um beispielsweise unter Berücksichtigung der Informationen über den Bestückungszustand Instrumente aus dem Sieb 10 zu entnehmen oder hinzuzufügen. Das gezeigte System umfasst auch ein chirurgisches Case-Cart 60, in dem weitere Instrumentensiebe 10 angeordnet sind. Das Case-Cart 60 ist mit einem fahrerlosen Transportfahrzeug 70 mittels Deichseln 61 und 71 gekoppelt. Das fahrerlose Transportfahrzeug 70 kann somit das Case-Cart 60 autonom bewegen, beispielsweise von einem Lager zu einem Operationssaal. Weiter ist eine Sterilisationsvorrichtung 50 vorgesehen, wie beispielsweise ein Autoklav, und die Steuereinrichtung 34 des Manipulators 30 ist eingerichtet, um beispielsweise Instrumentensiebe 10 in die Sterilisationsvorrichtung 50 zu bewegen und aus dieser wieder zu entnehmen. Die Bilderkennungseinheit 40 kann beispielsweise anhand der Silhouetten 14 erkennen, welche Instrumente an welchen Positionen angeordnet werden müssen. Alternativ oder zusätzlich kann die Bilderkennungseinheit 40 auch die verschiedenen maschinenlesbaren Codes 15 und 17 erfassen und entsprechend verarbeiten.

Vorzugsweise verfügt auch das Case-Cart 60 über maschinenlesbare Codes, sodass beispielsweise mit entsprechenden Bilderkennungsvorrichtungen ausgestattete Transportfahrzeuge 70 das Case-Cart 60 identifizieren können. Beispielsweise kann die Einrichtung der zentralen Sterilisationsabteilung einer Klinik über ein Lager an vollständig bestückten und sterilen Instrumentensieben verfügen, die bei Bedarf eingekoppelt werden können. Das Lager kann von einem Lagerroboter bedient werden und die Transportfahrzeuge 70 können automatisch die benötigten Case-Carts für spezifische Operationen abholen.

In einer bevorzugten Ausgestaltung werden die Instrumentensiebe in verschiedenen Konfigurationen mit verschiedenen chirurgischen Instrumenten bereitgestellt. Beispielsweise können Instrumentensiebe mit einer Basiskonfiguration bereitgestellt werden, die praktisch bei jeder Operation benötigt werden, während weitere spezielle Instrumentensiebe nur bestimmte Instrumente enthalten, die operationsabhängig verwendet werden.

### Bezugszeichenliste:

- 10: Instrumentensieb
- 11: Kopplungsvorrichtung
- 12: Behälter
- 14: Marker (Silhouette)
- 15: maschinenlesbarer Code
- 16: chirurgisches Instrument
- 17: maschinenlesbarer Code
- 18: alphanumerischer Code
- 20: Halteklemmen
- 21: Befestigungsstift
- 22,23: Federarme
- 30: Manipulator
- 32: Greifer
- 34: Steuereinrichtung des Manipulators
- 36: Tisch
- 40: Bilderkennungseinheit
- 41: Kamera
- 42: Einrichtung zur Bildverarbeitung
- 50: Sterilisationsvorrichtung
- 60: chirurgisches Case-Cart
- 61: Deichsel des Case-Carts
- 70: fahrerloses Transportfahrzeug
- 71: Deichsel des fahrerlosen Transportfahrzeug

## Patentansprüche

1. System umfassend ein Instrumentensieb umfassend einen Behälter (12) zur Aufnahme von chirurgischen Instrumenten (16), wobei der Behälter (12) mit eindeutigen visuellen Markern (14) für individuelle Instrumente versehen ist, um Instrumenten eindeutige Instrumenten-Positionen im Behälter zuzuordnen,
sowie einen Manipulator (30), eingerichtet zur Bestückung des Instrumentensiebs (10) mit chirurgischen Instrumenten (16) und/oder zur Entnahme von Instrumenten (16) aus dem Instrumentensieb und weiter umfassend eine Sterilisationsvorrichtung (50) und eine Steuereinrichtung (34) des Manipulators (30) mit einer Bilderkennungseinheit (40) mit einer Kamera (41) zur Bilderfassung und einer Einrichtung zur Bildverarbeitung (42), wobei die Bilderkennungseinheit (40) der Steuereinrichtung (34) des Manipulators (30) Informationen über den Bestückungszustand des Instrumentensiebs (10) übermittelt,
**dadurch gekennzeichnet, dass**
das System ein chirurgisches Case-Cart (60) umfasst, eingerichtet zur Aufnahme der Instrumentensiebe (10), und ein fahrerloses Transportfahrzeug (70) zum Bewegen des Case-Carts (60) und dass
das Instrumentensieb (10) Kopplungsvorrichtungen (11) aufweist, die eingerichtet sind, um das Instrumentensieb (10) in dem chirurgischen Case-Cart (60) anzuordnen und dass
die Steuereinrichtung des Manipulators (34) eingerichtet ist, um den Manipulator (30) unter Verwendung der Informationen zur Bestückung und/oder Entnahme von Instrumenten aus dem Instrumentensieb (10) anzusteuern und um bei Erkennung eines vollen oder im Wesentlichen vollen Bestückungszustands das Instrumentensieb (10) zu greifen und in die Sterilisationsvorrichtung (50) zu bewegen und nach Abschluss eines Sterilisationsvorgangs wieder zu entnehmen und in dem chirurgischen Case-Cart (60) anzuordnen.

2. System nach Anspruch 1, **dadurch gekennzeichnet, dass** die Marker (14) Silhouetten der individuellen Instrumente sind, wobei die Silhouetten vorzugsweise die gleiche Größe aufweisen wie die Instrumente, die sie repräsentieren.

3. System nach Anspruch 1 oder 2, **dadurch gekennzeichnet, dass** der Behälter (12) Halteklemmen (20) aufweist, die eingerichtet sind, um die Instrumente lösbar und mit Spiel zu halten.

4. System nach dem vorhergehenden Anspruch, **dadurch gekennzeichnet, dass** die Halteklemmen (12) Befestigungsstifte (21) aufweisen und der Behälter (12) Öffnungen, durch die die Stifte (21) geführt werden können, wobei die Stifte (21) zur Fixierung der Halteklemmen (20) am Behälter (12) verformbar eingerichtet sind.

5. System nach einem der Ansprüche 3 oder 4, **dadurch gekennzeichnet, dass** die Halteklemmen (20) jeweils zwei Federarme (22, 23) aufweisen, die eingerichtet sind, um zwischen sich einen Teil eines Instruments (16) lösbar und mit Spiel haltend aufzunehmen.

6. System nach einem der vorhergehenden Ansprüche, **dadurch gekennzeichnet, dass** das Instrumentensieb (10) autoklavierbar ist.

7. System nach einem der vorhergehenden Ansprüche, **dadurch gekennzeichnet, dass** die Instrumenten-Positionen im Behälter (12) in einer Ebene angeordnet sind, so dass sich die Instrumente (16) im Behälter (12) nicht überlappen.

8. Verfahren zum zumindest teilweise automatisierten Handhaben eines Instrumentensiebs in einem System nach einem der Ansprüche 1 bis 7, aufweisend folgende Schritte:
Bereitstellen eines derartigen Instrumentensiebs (10);
Bereitstellen eines Manipulators (30) mit einer zugeordneten Bilderkennungseinheit (40), umfassend eine Kamera (41) zur Bilderfassung und eine Einrichtung zur Bildverarbeitung (42);
Erfassen eines Bildes des Instrumentensiebes (10) und Erkennen des Bestückungszustands mittels der Bilderkennungseinheit (40);
Ansteuern des Manipulators (30) unter Berücksichtigung des Bestückungszustands zur Bestückung und/oder Entnahme von Instrumenten des Instrumentensiebs (10),
wobei bei Erkennung eines vollen oder im Wesentlichen vollen Bestückungszustands folgende Schritte ausgeführt werden:
Ergreifen des Instrumentensiebs (10) durch den Manipulator (30);
Bewegen des Instrumentensiebs (10) mittels des Manipulators (30) in eine Sterilisationsvorrichtung (50);
Durchführen eines Sterilisationsvorgangs in der Sterilisationsvorrichtung (50); und danach
Entnahme des sterilisierten Instrumentensiebs (10) mittels des Manipulators (30) aus der Sterilisationsvorrichtung (50) und
wobei nach der Entnahme des sterilisierten Instrumentensiebs mittels des Manipulators folgende Schritte durchgeführt werden:
Anordnen des Instrumentensiebs mittels des Manipulators in einen chirurgischen Case-Cart (60);
Bewegen des Case-Cart mittels eines fahrerlosen Transportfahrzeugs (70) zu einem Operationssaal.

9. Verfahren nach dem vorhergehenden Anspruch, **dadurch gekennzeichnet, dass** die Ansteuerung des Manipulators (30) derart erfolgt, dass der Manipulator (30) leere Instrumenten-Positionen bestückt.

10. Verfahren nach einem der vorhergehenden Verfahrensansprüche, **dadurch gekennzeichnet, dass** die Ansteuerung des Manipulators (30) derart erfolgt, dass der Manipulator (30) auf Anweisung eines Anwenders ausgewählte Instrumente (16) entnimmt.

## Claims

1. System comprising an instrument tray comprising a container (12) for receiving surgical instruments (16), wherein the container (12) is provided with dedicated visual markers (14) for individual instruments in order to assign instruments to dedicated instrument positions in the container, and a manipulator (30) configured to furnish the instrument tray (10) with surgical instruments (16) and/or remove instruments (16) from the instrument tray, and further comprising a sterilization device (50) and a control device (34) of the manipulator (30), having an image recognition unit (40) with a camera (41) for image capture and with an image processing device (42), wherein the image recognition unit (40) of the control device (34) of the manipulator (30) transmits information concerning the furnished state of the instrument tray (10),
**characterized in that**
the system comprises a surgical case cart (60) configured to receive the instrument tray (10), and a driverless transport vehicle (70) for moving the case cart (60), and **in that**
the instrument tray (10) has coupling devices (11) configured for arranging the instrument tray (10) in the surgical case cart (60), and **in that**
the manipulator control device (34) is configured to actuate the manipulator (30), using the information, to furnish and/or remove instruments from the instrument tray (10) and, when a fully or substantially fully furnished state is recognized, to grip the instrument tray (10) and move it into the sterilization device (50) and, after completion of a sterilization process, to remove it again and arrange it in the surgical case cart (60).

2. System according to Claim 1, **characterized in that** the markers (14) are silhouettes of the individual instruments, wherein the silhouettes are preferably the same size as the instruments that they represent.

3. System according to Claim 1 or 2, **characterized in that** the container (12) has holding clamps (20) which are configured to hold the instruments releasably and with play.

4. System according to the preceding claim, **characterized in that** the holding clamps (20) have fastening pins (21), and the container (12) has openings through which the pins (21) can be guided, wherein the pins (21) are configured deformably for fixing the holding clamps (20) on the container (12) .

5. System according to either of Claims 3 and 4, **characterized in that** the holding clamps (20) each have two spring arms (22, 23), which are configured to receive between them a part of an instrument (16) releasably and with play.

6. System according to one of the preceding claims, **characterized in that** the instrument tray (10) can be autoclaved.

7. System according to one of the preceding claims, **characterized in that** the instrument positions in the container (12) are arranged in one plane, such that the instruments (16) in the container (12) do not overlap.

8. Method for at least partially automated handling of an instrument tray in a system according to one of Claims 1 to 7, comprising the following steps:
making available such an instrument tray (10);
making available a manipulator (30) having an associated image recognition unit (40) comprising a camera (41) for image capture and an image processing device (42);
capturing an image of the instrument tray (10) and recognizing the furnished state by means of the image recognition unit (40);
actuating the manipulator (30), in consideration of the furnished state, so as to furnish and/or remove instruments from the instrument tray (10),
wherein, when a fully or substantially fully furnished state is recognized, the following steps are carried out:
gripping of the instrument tray (10) by the manipulator (30);
movement of the instrument tray (10) into a sterilization device (50) by means of the manipulator (30);
performance of a sterilization procedure in the sterilization device (50); and then
removal of the sterilized instrument tray (10) from the sterilization device (50) by means of the manipulator (30), and
wherein, after removal of the sterilized instrument tray by means of the manipulator, the following steps are carried out:
placement of the instrument tray into a surgical case cart (60) by means of the manipulator;
movement of the case cart to an operating theatre by means of a driverless transport vehicle (70).

9. Method according to the preceding claim, **characterized in that** the actuation of the manipulator (30) takes place in such a way that the manipulator (30) furnishes empty instrument positions.

10. Method according to one of the preceding method claims, **characterized in that** the actuation of the manipulator (30) takes place in such a way that the manipulator (30) removes selected instruments (16) on the instructions of a user.

## Revendications

1. Système comprenant un plateau à instruments comprenant un récipient (12) destiné à recevoir des instruments chirurgicaux (16), le récipient (12) étant pourvu de marqueurs visuels uniques (14) pour des instruments individuels afin d'attribuer aux instruments des positions d'instruments uniques dans le récipient, ainsi qu'un manipulateur (30), adapté pour charger le plateau à instruments (10) avec des instruments chirurgicaux (16) et/ou pour retirer des instruments (16) du plateau à instruments et comprenant en outre un dispositif de stérilisation (50) et un appareil de commande (34) du manipulateur (30) avec une unité de reconnaissance d'images (40) avec une caméra (41) pour l'acquisition d'images et un appareil pour le traitement d'images (42), l'unité de reconnaissance d'images (40) transmettant à l'appareil de commande (34) du manipulateur (30) des informations sur l'état de chargement du plateau à instruments (10),
**caractérisé en ce que**
le système comprend un chariot de cas chirurgical (60) adaptée pour recevoir les plateaux à instruments (10) et un véhicule de transport sans conducteur (70) pour déplacer le chariot de cas (60), et **en ce que**
le plateau à instruments (10) présente des dispositifs d'accouplement (11) adaptés pour agencer le plateau à instruments (10) dans le chariot de cas chirurgical (60) et **en ce que**
l'appareil de commande du manipulateur (34) est adapté pour commander le manipulateur (30) en utilisant les informations pour le chargement et/ou le retrait d'instruments du plateau à instruments (10) et pour, lors de la détection d'un état de chargement complet ou essentiellement complet, saisir le plateau à instruments (10) et le déplacer dans le dispositif de stérilisation (50) et, à la fin d'une opération de stérilisation, le retirer à nouveau et l'agencer dans le chariot de cas chirurgical (60).

2. Système selon la revendication 1, **caractérisé en ce que** les marqueurs (14) sont des silhouettes des instruments individuels, les silhouettes présentant de préférence la même taille que les instruments qu'elles représentent.

3. Système selon la revendication 1 ou 2, **caractérisé en ce que** le récipient (12) présente des pinces de maintien (20) adaptées pour maintenir les instruments de manière amovible et avec du jeu.

4. Système selon la revendication précédente, **caractérisé en ce que** les pinces de maintien (12) présentent des broches de fixation (21) et le récipient (12) présente des ouvertures à travers lesquelles les broches (21) peuvent être guidées, les broches (21) étant adaptées pour pouvoir être déformées afin de fixer les pinces de maintien (20) au récipient (12).

5. Système selon l'une quelconque des revendications 3 ou 4, **caractérisé en ce que** les pinces de maintien (20) présentent chacune deux bras élastiques (22, 23) adaptés pour recevoir entre eux, de manière amovible et avec jeu, une partie d'un instrument (16).

6. Système selon l'une quelconque des revendications précédentes, **caractérisé en ce que** le plateau à instruments (10) est autoclavable.

7. Système selon l'une quelconque des revendications précédentes, **caractérisé en ce que** les positions d'instruments dans le récipient (12) sont agencées dans un plan tel que les instruments (16) ne se chevauchent pas dans le récipient (12).

8. Procédé pour la manipulation au moins partiellement automatisée d'un plateau à instruments dans un système selon l'une quelconque des revendications 1 à 7, présentant les étapes suivantes :
la fourniture d'un tel plateau à instruments (10) ;
la fourniture d'un manipulateur (30) avec une unité de reconnaissance d'images associée (40), comprenant une caméra (41) pour l'acquisition d'images et un appareil pour le traitement d'images (42) ;
l'acquisition d'une image du plateau à instruments (10) et la détection de l'état de chargement au moyen de l'unité de reconnaissance d'images (40) ;
la commande du manipulateur (30) en tenant compte de l'état de chargement pour le chargement et/ou le retrait d'instruments du plateau à instruments (10), les étapes suivantes étant exécutées lors de la détection d'un état de chargement complet ou essentiellement complet :
la saisie du plateau à instruments (10) par le manipulateur (30) ;
le déplacement du plateau à instruments (10) au moyen du manipulateur (30) dans un dispositif de stérilisation (50) ;
la réalisation d'une opération de stérilisation dans le dispositif de stérilisation (50) ; et ensuite
le retrait du plateau à instruments stérilisé (10) du dispositif de stérilisation (50) au moyen du manipulateur (30), et
après le retrait du plateau à instruments stérilisé au moyen du manipulateur, les étapes suivantes étant réalisées :
l'agencement du plateau à instruments dans un chariot de cas chirurgical (60) au moyen du manipulateur ;
le déplacement du chariot de cas vers une salle d'opération au moyen d'un véhicule de transport sans conducteur (70).

9. Procédé selon la revendication précédente, **caractérisé en ce que** la commande du manipulateur (30) est effectuée de telle sorte que le manipulateur (30) charge des positions d'instruments vides.

10. Procédé selon l'une quelconque des revendications précédentes, **caractérisé en ce que** la commande du manipulateur (30) est effectuée de telle sorte que le manipulateur (30) retire des instruments sélectionnés (16) sur instruction d'un utilisateur.
